# EUROPEAN PATENT APPLICATION

(11) **EP 4 056 595 A1**
(43) Date of publication of application: **14.09.2022**
(21) Application number: 21177472.4
(22) Date of filing: 02.06.2021
(51) Int. Cl.: C07K 16/40

(54) **MONOCLONAL ANTIBODIES AGAINST COMMON CARP BETA-ENOLASE**

(30) Priority: 12.03.2021 LT 2021509
(71) Applicant: Vilnius University, 01513 Vilnius (LT)
(72) Inventor: Zvirblis, Gintautas, Vilnius (LT); Sliziene, Aiste, Vilnius (LT); Pleckaityte, Milda, Vilnius (LT); Zaveckas, Mindaugas, Vilnius (LT)
(74) Representative: Petniunaite, Jurga

(57) **Abstract**

The invention belongs to the field of protein biotechnology. New monoclonal antibodies against carp β-enolase and hybridoma cells producing them are described herein. Monoclonal antibodies can be used to detect carp and other fish proteins in foods, and to identify commercial carp extracts.

SEQUENCE LISTING

<110>  Vilnius University

<120>  MONOCLONAL ANTIBODIES AGAINST COMMON CARP BETA-ENOLASE

<130>  V83-80

<160>  12

<170>  PatentIn version 3.5

<210>  1
<211>  433
<212>  PRT
<213>  Cyprinus carpio

<400>  1

Met Ser Ile Ser Lys Ile His Ala Arg Glu Ile Leu Asp Ser Arg Gly
1               5                   10                  15

Asn Pro Thr Val Glu Val Asp Leu Tyr Thr Ala Lys Gly Arg Phe Arg
            20                  25                  30

Ala Ala Val Pro Ser Gly Ala Ser Thr Gly Val His Glu Ala Leu Glu
        35                  40                  45

Leu Arg Asp Gly Asp Lys Thr Arg Tyr Leu Gly Lys Gly Thr Gln Lys
    50                  55                  60

Ala Val Asp His Val Asn Lys Glu Ile Ala Pro Lys Leu Ile Glu Lys
65                  70                  75                  80

Lys Phe Ser Val Val Glu Gln Glu Lys Ile Asp Lys Phe Met Leu Glu
                85                  90                  95

Leu Asp Gly Thr Glu Asn Lys Ser Lys Phe Gly Ala Asn Ala Ile Leu
            100                 105                 110

Gly Val Ser Leu Ala Val Cys Lys Ala Gly Ala Ala Glu Lys Gly Val
        115                 120                 125

Pro Leu Tyr Arg His Ile Ala Asp Leu Ala Gly Asn Lys Asp Val Ile
    130                 135                 140

Leu Pro Val Pro Ala Phe Asn Val Ile Asn Gly Gly Ser His Ala Gly
145                 150                 155                 160

Asn Lys Leu Ala Met Gln Glu Phe Met Ile Leu Pro Val Gly Ala Lys
                165                 170                 175

Asn Phe His Glu Ala Met Arg Ile Gly Ala Glu Val Tyr His Asn Leu
            180                 185                 190

Lys Asn Val Ile Lys Ala Lys Tyr Gly Lys Asp Ala Thr Asn Val Gly
        195                 200                 205

Asp Glu Gly Gly Phe Ala Pro Asn Ile Leu Glu Asn Asn Glu Ala Leu
    210                 215                 220

Glu Leu Leu Lys Ser Ala Ile Glu Lys Ala Gly Tyr Pro Asp Lys Ile
225                 230                 235                 240

Ile Ile Gly Met Asp Val Ala Ala Ser Glu Phe Phe Lys Asn Gly Lys
                245                 250                 255

Tyr Asp Leu Asp Phe Lys Ser Pro Asp Asp Pro Lys Arg His Ile Thr
            260                 265                 270

Gly Asp Gln Leu Gly Asp Leu Tyr Lys Ser Phe Ile Lys Asn Tyr Pro
        275                 280                 285

Val Gln Ser Ile Glu Asp Pro Phe Asp Gln Asp Asp Trp Glu Asn Trp
    290                 295                 300

Ser Lys Phe Thr Gly Ser Val Asp Ile Gln Val Val Gly Asp Asp Leu
305                 310                 315                 320

Thr Val Thr Asn Pro Lys Arg Ile Gln Gln Ala Cys Glu Lys Lys Ala
                325                 330                 335

Cys Asn Cys Leu Leu Leu Lys Val Asn Gln Ile Gly Ser Val Thr Glu
            340                 345                 350

Ser Ile Gln Ala Cys Lys Leu Ala Gln Ser Asn Gly Trp Gly Val Met
        355                 360                 365

Val Ser His Arg Ser Gly Glu Thr Glu Asp Thr Phe Ile Ala Asp Leu
    370                 375                 380

Val Val Gly Leu Cys Thr Gly Gln Ile Lys Thr Gly Ala Pro Cys Arg
385                 390                 395                 400

Ser Glu Arg Leu Ala Lys Tyr Asn Gln Leu Met Arg Ile Glu Glu Glu
                405                 410                 415

Leu Gly Asp Lys Ala Lys Phe Ala Gly Lys Asp Phe Arg His Pro Lys
            420                 425                 430

Leu

<210> 2
<211> 155
<212> PRT
<213> Cyprinus carpio

<400> 2

Lys Asp Val Ile Leu Pro Val Pro Ala Phe Asn Val Ile Asn Gly Gly
1               5                   10                  15

Ser His Ala Gly Asn Lys Leu Ala Met Gln Glu Phe Met Ile Leu Pro
            20                  25                  30

Val Gly Ala Lys Asn Phe His Glu Ala Met Arg Ile Gly Ala Glu Val
        35                  40                  45

Tyr His Asn Leu Lys Asn Val Ile Lys Ala Lys Tyr Gly Lys Asp Ala
    50                  55                  60

Thr Asn Val Gly Asp Glu Gly Gly Phe Ala Pro Asn Ile Leu Glu Asn
65                  70                  75                  80

Asn Glu Ala Leu Glu Leu Leu Lys Ser Ala Ile Glu Lys Ala Gly Tyr
                85                  90                  95

Pro Asp Lys Ile Ile Ile Gly Met Asp Val Ala Ala Ser Glu Phe Phe
            100                 105                 110

Lys Asn Gly Lys Tyr Asp Leu Asp Phe Lys Ser Pro Asp Asp Pro Lys
        115                 120                 125

Arg His Ile Thr Gly Asp Gln Leu Gly Asp Leu Tyr Lys Ser Phe Ile
    130                 135                 140

Lys Asn Tyr Pro Val Gln Ser Ile Glu Asp Pro
145                 150                 155

<210> 3
<211> 33
<212> DNA
<213> Artificial Sequence

<220>
<223> Primer1

<400> 3
gagatctatg tccatcagta agattcacgc tcg                                33

<210>  4
<211>  28
<212>  DNA
<213>  Artificial Sequence

<220>
<223>  Primer2

<400>  4
cgtcgactca gagtttgggg tggcggaa                                          28


<210>  5
<211>  23
<212>  DNA
<213>  Artificial Sequence

<220>
<223>  Primer3

<400>  5
cgactcacta tagggagagc ggc                                               23


<210>  6
<211>  24
<212>  DNA
<213>  Artificial Sequence

<220>
<223>  Primer4

<400>  6
aagaacatcg attttccatg gcag                                              24


<210>  7
<211>  115
<212>  PRT
<213>  Mus musculus

<400>  7

Gln Val Gln Leu Gln Gln Ser Gly Ala Glu Leu Val Arg Pro Gly Ala
1               5                   10                  15

Ser Val Thr Leu Ser Cys Lys Ala Ser Gly Tyr Thr Phe Ile Asp Tyr
            20                  25                  30

Glu Met His Trp Val Lys Gln Thr Pro Val His Gly Leu Glu Trp Ile
        35                  40                  45

Gly Ala Ile Asp Pro Glu Thr Gly Gly Thr Ala Tyr Asn Gln Lys Phe
    50                  55                  60

Lys Gly Lys Ala Ile Leu Thr Ala Asp Lys Ser Ser Ser Thr Ala Tyr
65                  70                  75                  80

Met Glu Leu Arg Ser Leu Thr Ser Glu Asp Ser Ala Val Tyr Tyr Cys
                85                  90                  95

Ser Pro Thr Leu Gln Gly Asp Tyr Trp Gly Gln Gly Thr Thr Leu Thr
            100                 105                 110

Val Ser Ser
        115

<210> 8
<211> 112
<212> PRT
<213> Mus musculus

<400> 8

Asp Val Val Met Thr Gln Thr Pro Leu Ser Leu Pro Val Ser Leu Gly
1               5                   10                  15

Asp Gln Ala Ser Ile Ser Cys Arg Ser Ser Gln Ser Leu Val His Ser
            20                  25                  30

Asp Gly Asn Thr Tyr Leu Tyr Trp Tyr Leu Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Phe Cys Ser Gln Ser
                85                  90                  95

Thr His Ile Pro Trp Thr Phe Gly Gly Gly Thr Lys Val Glu Ile Lys
            100                 105                 110

<210> 9
<211> 836
<212> PRT
<213> Escherichia coli

<400> 9

Met Gly Ser Ser His His His His His His Ser Ser Gly Leu Val Pro
1               5                   10                  15

Arg Gly Ser His Met Ala Ser Gly Lys Ile Glu Glu Gly Lys Leu Val
            20                  25                  30

Ile Trp Ile Asn Gly Asp Lys Gly Tyr Asn Gly Leu Ala Glu Val Gly
        35                  40                  45

Lys Lys Phe Glu Lys Asp Thr Gly Ile Lys Val Thr Val Glu His Pro
    50                  55                  60

Asp Lys Leu Glu Glu Lys Phe Pro Gln Val Ala Ala Thr Gly Asp Gly
65                  70                  75                  80

Pro Asp Ile Ile Phe Trp Ala His Asp Arg Phe Gly Gly Tyr Ala Gln
                85                  90                  95

Ser Gly Leu Leu Ala Glu Ile Thr Pro Asp Lys Ala Phe Gln Asp Lys
            100                 105                 110

Leu Tyr Pro Phe Thr Trp Asp Ala Val Arg Tyr Asn Gly Lys Leu Ile
        115                 120                 125

Ala Tyr Pro Ile Ala Val Glu Ala Leu Ser Leu Ile Tyr Asn Lys Asp
    130                 135                 140

Leu Leu Pro Asn Pro Pro Lys Thr Trp Glu Glu Ile Pro Ala Leu Asp
145                 150                 155                 160

Lys Glu Leu Lys Ala Lys Gly Lys Ser Ala Leu Met Phe Asn Leu Gln
                165                 170                 175

Glu Pro Tyr Phe Thr Trp Pro Leu Ile Ala Ala Asp Gly Gly Tyr Ala
            180                 185                 190

Phe Lys Tyr Glu Asn Gly Lys Tyr Asp Ile Lys Asp Val Gly Val Asp
        195                 200                 205

Asn Ala Gly Ala Lys Ala Gly Leu Thr Phe Leu Val Asp Leu Ile Lys
    210                 215                 220

Asn Lys His Met Asn Ala Asp Thr Asp Tyr Ser Ile Ala Glu Ala Ala
225                 230                 235                 240

Phe Asn Lys Gly Glu Thr Ala Met Thr Ile Asn Gly Pro Trp Ala Trp
                245                 250                 255

Ser Asn Ile Asp Thr Ser Lys Val Asn Tyr Gly Val Thr Val Leu Pro
            260                 265                 270

Thr Phe Lys Gly Gln Pro Ser Lys Pro Phe Val Gly Val Leu Ser Ala
        275                 280                 285

Gly Ile Asn Ala Ala Ser Pro Asn Lys Glu Leu Ala Lys Glu Phe Leu
    290                 295                 300

Glu Asn Tyr Leu Leu Thr Asp Glu Gly Leu Glu Ala Val Asn Lys Asp
305                 310                 315                 320

Lys Pro Leu Gly Ala Val Ala Leu Lys Ser Tyr Glu Glu Glu Leu Ala
                325                 330                 335

Lys Asp Pro Arg Ile Ala Ala Thr Met Glu Asn Ala Gln Lys Gly Glu
            340                 345                 350

Ile Met Pro Asn Ile Pro Gln Met Ser Ala Phe Trp Tyr Ala Val Arg
        355                 360                 365

Thr Ala Val Ile Asn Ala Ala Ser Gly Arg Gln Thr Val Asp Glu Ala
    370                 375                 380

Leu Lys Asp Ala Gln Thr Arg Ile Thr Lys Gly Glu Asn Leu Tyr Phe
385                 390                 395                 400

Gln Gly Ser Met Ser Ile Ser Lys Ile His Ala Arg Glu Ile Leu Asp
                405                 410                 415

Ser Arg Gly Asn Pro Thr Val Glu Val Asp Leu Tyr Thr Ala Lys Gly
            420                 425                 430

Arg Phe Arg Ala Ala Val Pro Ser Gly Ala Ser Thr Gly Val His Glu
        435                 440                 445

Ala Leu Glu Leu Arg Asp Gly Asp Lys Thr Arg Tyr Leu Gly Lys Gly
    450                 455                 460

Thr Gln Lys Ala Val Asp His Val Asn Lys Glu Ile Ala Pro Lys Leu
465                 470                 475                 480

Ile Glu Lys Lys Phe Ser Val Val Glu Gln Glu Lys Ile Asp Lys Phe
                485                 490                 495

Met Leu Glu Leu Asp Gly Thr Glu Asn Lys Ser Lys Phe Gly Ala Asn
            500                 505                 510

Ala Ile Leu Gly Val Ser Leu Ala Val Cys Lys Ala Gly Ala Ala Glu
        515                 520                 525

Lys Gly Val Pro Leu Tyr Arg His Ile Ala Asp Leu Ala Gly Asn Lys
    530                 535                 540

Asp Val Ile Leu Pro Val Pro Ala Phe Asn Val Ile Asn Gly Gly Ser
545                 550                 555                 560

His Ala Gly Asn Lys Leu Ala Met Gln Glu Phe Met Ile Leu Pro Val
                565                 570                 575

Gly Ala Lys Asn Phe His Glu Ala Met Arg Ile Gly Ala Glu Val Tyr
            580                 585                 590

His Asn Leu Lys Asn Val Ile Lys Ala Lys Tyr Gly Lys Asp Ala Thr
        595                 600                 605

Asn Val Gly Asp Glu Gly Gly Phe Ala Pro Asn Ile Leu Glu Asn Asn
    610                 615                 620

Glu Ala Leu Glu Leu Leu Lys Ser Ala Ile Glu Lys Ala Gly Tyr Pro
625                 630                 635                 640

Asp Lys Ile Ile Ile Gly Met Asp Val Ala Ala Ser Glu Phe Phe Lys
                645                 650                 655

Asn Gly Lys Tyr Asp Leu Asp Phe Lys Ser Pro Asp Asp Pro Lys Arg
            660                 665                 670

His Ile Thr Gly Asp Gln Leu Gly Asp Leu Tyr Lys Ser Phe Ile Lys
        675                 680                 685

Asn Tyr Pro Val Gln Ser Ile Glu Asp Pro Phe Asp Gln Asp Asp Trp
    690                 695                 700

Glu Asn Trp Ser Lys Phe Thr Gly Ser Val Asp Ile Gln Val Val Gly
705                 710                 715                 720

Asp Asp Leu Thr Val Thr Asn Pro Lys Arg Ile Gln Gln Ala Cys Glu
                725                 730                 735

Lys Lys Ala Cys Asn Cys Leu Leu Leu Lys Val Asn Gln Ile Gly Ser
            740                 745                 750

Val Thr Glu Ser Ile Gln Ala Cys Lys Leu Ala Gln Ser Asn Gly Trp
        755                 760                 765

Gly Val Met Val Ser His Arg Ser Gly Glu Thr Glu Asp Thr Phe Ile
    770                 775                 780

Ala Asp Leu Val Val Gly Leu Cys Thr Gly Gln Ile Lys Thr Gly Ala

785                 790                 795                 800

Pro Cys Arg Ser Glu Arg Leu Ala Lys Tyr Asn Gln Leu Met Arg Ile
                805                 810                 815

Glu Glu Glu Leu Gly Asp Lys Ala Lys Phe Ala Gly Lys Asp Phe Arg
            820                 825                 830

His Pro Lys Leu
        835

<210>  10
<211>  698
<212>  PRT
<213>  Escherichia coli

<400>  10

Met Gly Ser Ser His His His His His His Ser Ser Gly Leu Val Pro
1               5                   10                  15

Arg Gly Ser His Met Ala Ser Gly Lys Ile Glu Glu Gly Lys Leu Val
            20                  25                  30

Ile Trp Ile Asn Gly Asp Lys Gly Tyr Asn Gly Leu Ala Glu Val Gly
        35                  40                  45

Lys Lys Phe Glu Lys Asp Thr Gly Ile Lys Val Thr Val Glu His Pro
    50                  55                  60

Asp Lys Leu Glu Glu Lys Phe Pro Gln Val Ala Ala Thr Gly Asp Gly
65                  70                  75                  80

Pro Asp Ile Ile Phe Trp Ala His Asp Arg Phe Gly Gly Tyr Ala Gln
                85                  90                  95

Ser Gly Leu Leu Ala Glu Ile Thr Pro Asp Lys Ala Phe Gln Asp Lys
            100                 105                 110

Leu Tyr Pro Phe Thr Trp Asp Ala Val Arg Tyr Asn Gly Lys Leu Ile
        115                 120                 125

Ala Tyr Pro Ile Ala Val Glu Ala Leu Ser Leu Ile Tyr Asn Lys Asp
    130                 135                 140

Leu Leu Pro Asn Pro Pro Lys Thr Trp Glu Glu Ile Pro Ala Leu Asp
145                 150                 155                 160

Lys Glu Leu Lys Ala Lys Gly Lys Ser Ala Leu Met Phe Asn Leu Gln

                165                 170                 175

Glu Pro Tyr Phe Thr Trp Pro Leu Ile Ala Ala Asp Gly Gly Tyr Ala
            180                 185                 190

Phe Lys Tyr Glu Asn Gly Lys Tyr Asp Ile Lys Asp Val Gly Val Asp
        195                 200                 205

Asn Ala Gly Ala Lys Ala Gly Leu Thr Phe Leu Val Asp Leu Ile Lys
    210                 215                 220

Asn Lys His Met Asn Ala Asp Thr Asp Tyr Ser Ile Ala Glu Ala Ala
225                 230                 235                 240

Phe Asn Lys Gly Glu Thr Ala Met Thr Ile Asn Gly Pro Trp Ala Trp
                245                 250                 255

Ser Asn Ile Asp Thr Ser Lys Val Asn Tyr Gly Val Thr Val Leu Pro
            260                 265                 270

Thr Phe Lys Gly Gln Pro Ser Lys Pro Phe Val Gly Val Leu Ser Ala
        275                 280                 285

Gly Ile Asn Ala Ala Ser Pro Asn Lys Glu Leu Ala Lys Glu Phe Leu
    290                 295                 300

Glu Asn Tyr Leu Leu Thr Asp Glu Gly Leu Glu Ala Val Asn Lys Asp
305                 310                 315                 320

Lys Pro Leu Gly Ala Val Ala Leu Lys Ser Tyr Glu Glu Glu Leu Ala
                325                 330                 335

Lys Asp Pro Arg Ile Ala Ala Thr Met Glu Asn Ala Gln Lys Gly Glu
            340                 345                 350

Ile Met Pro Asn Ile Pro Gln Met Ser Ala Phe Trp Tyr Ala Val Arg
        355                 360                 365

Thr Ala Val Ile Asn Ala Ala Ser Gly Arg Gln Thr Val Asp Glu Ala
    370                 375                 380

Leu Lys Asp Ala Gln Thr Arg Ile Thr Lys Gly Glu Asn Leu Tyr Phe
385                 390                 395                 400

Gln Gly Ser Met Ser Ile Ser Lys Ile His Ala Arg Glu Ile Leu Asp
                405                 410                 415

Ser Arg Gly Asn Pro Thr Val Glu Val Asp Leu Tyr Thr Ala Lys Gly
            420                 425                 430

Arg Phe Arg Ala Ala Val Pro Ser Gly Ala Ser Thr Gly Val His Glu
        435                 440                 445

Ala Leu Glu Leu Arg Asp Gly Asp Lys Thr Arg Tyr Leu Gly Lys Gly
    450                 455                 460

Thr Gln Lys Ala Val Asp His Val Asn Lys Glu Ile Ala Pro Lys Leu
465                 470                 475                 480

Ile Glu Lys Lys Phe Ser Val Val Glu Gln Glu Lys Ile Asp Lys Phe
                485                 490                 495

Met Leu Glu Leu Asp Gly Thr Glu Asn Lys Ser Lys Phe Gly Ala Asn
            500                 505                 510

Ala Ile Leu Gly Val Ser Leu Ala Val Cys Lys Ala Gly Ala Ala Glu
        515                 520                 525

Lys Gly Val Pro Leu Tyr Arg His Ile Ala Asp Leu Ala Gly Asn Lys
    530                 535                 540

Asp Val Ile Leu Pro Val Pro Ala Phe Asn Val Ile Asn Gly Gly Ser
545                 550                 555                 560

His Ala Gly Asn Lys Leu Ala Met Gln Glu Phe Met Ile Leu Pro Val
                565                 570                 575

Gly Ala Lys Asn Phe His Glu Ala Met Arg Ile Gly Ala Glu Val Tyr
            580                 585                 590

His Asn Leu Lys Asn Val Ile Lys Ala Lys Tyr Gly Lys Asp Ala Thr
        595                 600                 605

Asn Val Gly Asp Glu Gly Gly Phe Ala Pro Asn Ile Leu Glu Asn Asn
    610                 615                 620

Glu Ala Leu Glu Leu Leu Lys Ser Ala Ile Glu Lys Ala Gly Tyr Pro
625                 630                 635                 640

Asp Lys Ile Ile Ile Gly Met Asp Val Ala Ala Ser Glu Phe Phe Lys
                645                 650                 655

Asn Gly Lys Tyr Asp Leu Asp Phe Lys Ser Pro Asp Asp Pro Lys Arg
            660                 665                 670

His Ile Thr Gly Asp Gln Leu Gly Asp Leu Tyr Lys Ser Phe Ile Lys
        675                 680                 685

Asn Tyr Pro Val Gln Ser Ile Glu Asp Pro
    690                 695

<210>  11
<211>  543
<212>  PRT
<213>  Escherichia coli

<400>  11

Met Gly Ser Ser His His His His His His Ser Ser Gly Leu Val Pro
1               5                   10                  15

Arg Gly Ser His Met Ala Ser Gly Lys Ile Glu Glu Gly Lys Leu Val
            20                  25                  30

Ile Trp Ile Asn Gly Asp Lys Gly Tyr Asn Gly Leu Ala Glu Val Gly
        35                  40                  45

Lys Lys Phe Glu Lys Asp Thr Gly Ile Lys Val Thr Val Glu His Pro
    50                  55                  60

Asp Lys Leu Glu Glu Lys Phe Pro Gln Val Ala Ala Thr Gly Asp Gly
65                  70                  75                  80

Pro Asp Ile Ile Phe Trp Ala His Asp Arg Phe Gly Gly Tyr Ala Gln
                85                  90                  95

Ser Gly Leu Leu Ala Glu Ile Thr Pro Asp Lys Ala Phe Gln Asp Lys
            100                 105                 110

Leu Tyr Pro Phe Thr Trp Asp Ala Val Arg Tyr Asn Gly Lys Leu Ile
        115                 120                 125

Ala Tyr Pro Ile Ala Val Glu Ala Leu Ser Leu Ile Tyr Asn Lys Asp
    130                 135                 140

Leu Leu Pro Asn Pro Pro Lys Thr Trp Glu Glu Ile Pro Ala Leu Asp
145                 150                 155                 160

Lys Glu Leu Lys Ala Lys Gly Lys Ser Ala Leu Met Phe Asn Leu Gln
                165                 170                 175

Glu Pro Tyr Phe Thr Trp Pro Leu Ile Ala Ala Asp Gly Gly Tyr Ala
            180                 185                 190

Phe Lys Tyr Glu Asn Gly Lys Tyr Asp Ile Lys Asp Val Gly Val Asp
        195                 200                 205

Asn Ala Gly Ala Lys Ala Gly Leu Thr Phe Leu Val Asp Leu Ile Lys
    210                 215                 220

Asn Lys His Met Asn Ala Asp Thr Asp Tyr Ser Ile Ala Glu Ala Ala
225                 230                 235                 240

Phe Asn Lys Gly Glu Thr Ala Met Thr Ile Asn Gly Pro Trp Ala Trp
                245                 250                 255

Ser Asn Ile Asp Thr Ser Lys Val Asn Tyr Gly Val Thr Val Leu Pro
            260                 265                 270

Thr Phe Lys Gly Gln Pro Ser Lys Pro Phe Val Gly Val Leu Ser Ala
        275                 280                 285

Gly Ile Asn Ala Ala Ser Pro Asn Lys Glu Leu Ala Lys Glu Phe Leu
    290                 295                 300

Glu Asn Tyr Leu Leu Thr Asp Glu Gly Leu Glu Ala Val Asn Lys Asp
305                 310                 315                 320

Lys Pro Leu Gly Ala Val Ala Leu Lys Ser Tyr Glu Glu Glu Leu Ala
                325                 330                 335

Lys Asp Pro Arg Ile Ala Ala Thr Met Glu Asn Ala Gln Lys Gly Glu
            340                 345                 350

Ile Met Pro Asn Ile Pro Gln Met Ser Ala Phe Trp Tyr Ala Val Arg
        355                 360                 365

Thr Ala Val Ile Asn Ala Ala Ser Gly Arg Gln Thr Val Asp Glu Ala
    370                 375                 380

Leu Lys Asp Ala Gln Thr Arg Ile Thr Lys Gly Glu Asn Leu Tyr Phe
385                 390                 395                 400

Gln Gly Ser Met Ser Ile Ser Lys Ile His Ala Arg Glu Ile Leu Asp
                405                 410                 415

Ser Arg Gly Asn Pro Thr Val Glu Val Asp Leu Tyr Thr Ala Lys Gly
            420                 425                 430

Arg Phe Arg Ala Ala Val Pro Ser Gly Ala Ser Thr Gly Val His Glu
        435                 440                 445

Ala Leu Glu Leu Arg Asp Gly Asp Lys Thr Arg Tyr Leu Gly Lys Gly
    450                 455                 460

Thr Gln Lys Ala Val Asp His Val Asn Lys Glu Ile Ala Pro Lys Leu
465                 470                 475                 480

Ile Glu Lys Lys Phe Ser Val Val Glu Gln Glu Lys Ile Asp Lys Phe
                485                 490                 495

Met Leu Glu Leu Asp Gly Thr Glu Asn Lys Ser Lys Phe Gly Ala Asn
            500                 505                 510

Ala Ile Leu Gly Val Ser Leu Ala Val Cys Lys Ala Gly Ala Ala Glu
        515                 520                 525

Lys Gly Val Pro Leu Tyr Arg His Ile Ala Asp Leu Ala Gly Asn
    530                 535                 540

<210>  12
<211>  155
<212>  PRT
<213>  Cyprinus carpio

<400>  12

Lys Asp Val Ile Leu Pro Val Pro Ala Phe Asn Val Ile Asn Gly Gly
1               5                   10                  15

Ser His Ala Gly Asn Lys Leu Ala Met Gln Glu Phe Met Ile Leu Pro
            20                  25                  30

Val Gly Ala Lys Asn Phe His Glu Ala Met Arg Ile Gly Ala Glu Val
        35                  40                  45

Tyr His Asn Leu Lys Asn Val Ile Lys Ala Lys Tyr Gly Lys Asp Ala
    50                  55                  60

Thr Asn Val Gly Asp Glu Gly Gly Phe Ala Pro Asn Ile Leu Glu Asn
65                  70                  75                  80

Asn Glu Ala Leu Glu Leu Leu Lys Ser Ala Ile Glu Lys Ala Gly Tyr
                85                  90                  95

Pro Asp Lys Ile Ile Ile Gly Met Asp Val Ala Ala Ser Glu Phe Phe
            100                 105                 110

Lys Asn Gly Lys Tyr Asp Leu Asp Phe Lys Ser Pro Asp Asp Pro Lys
        115                 120                 125

Arg His Ile Thr Gly Asp Gln Leu Gly Asp Leu Tyr Lys Ser Phe Ile
    130                 135                 140

Lys Asn Tyr Pro Val Gln Ser Ile Glu Asp Pro
145                 150                 155

## Description

### Field of the Invention

This invention belongs to the field of biotechnology; it describes the generation of new monoclonal antibodies that are specific to common carp β-enolase.

### Background of the Invention

Enolase (phosphopyruvate hydratase, EC 4.2.1.11) is a metalloenzyme, involved in metabolism of glucose by catalysing the dehydration of 2-phosphoglycerate to phosphoenolpyruvate [Nakagawa and Nagayama. Comp. Biochem. Physiol. 1991, 98B, pp. 355-359; Diaz-Ramos et al. J Biomed Biotechnol. 2012]. This enzyme is found in microorganisms and mammals, and its amino acid sequence identity among different species is more than 50% [Witkowska et al. FEMS Immunol Med Microbiol. 2005, 45, pp. 53-62]. Enolase is purified and analysed from variety of organisms: from muscle of human, chicken, rabbit, rainbow trout, rat; from yeast: *Saccharomyces cerevisiae;* from plants: potato and spinach and from different bacteria: *Escherichia coli, Chloroflexus aurantiacus, Thermus aquaticus* and etc [Van der Straeten et al. Plant Cell. 1991, 3, pp. 719-735; Nakagawa and Nagayama. Comp. Biochem. Physiol. 1991, 98B, pp. 355-359; Zadvornyy et al. Front Bioeng Biotechnol. 2015, 3]. In vertebrates enolase is active as dimer (subunits molecular weight (MW) about 50 kDa) and is found in three isoforms: α, β and γ [Kleine-Tebbe and Jakob, Springer. 2015, pp.383-384]. α-enolase (Eno1) is expressed in most tissues, β-enolase (Eno3) mainly found in muscle tissues and γ-enolase (Eno2) is detected in neuron and neuroendocrine tissues [Díaz-Ramos et al. J Biomed Biotechnol. 2012]. β-enolase was first time purified and characterized from common carp in 1983 [Pietkiewicz et al. Comp. Biochem. Physiol. 1983, 75B, pp. 693-698].

Fish is one of the main food allergens that causes allergy to less than 1% of the general population. It is known that patients can be allergic to one specific fish or sensitive to several different fish species. Allergy symptoms (urticaria, diarrhoea, vomiting, bronchioconstriction and etc.) can affect patient skin, respiratory tract and digestive tract not only after eating fish but also during fish meals preparation. In some cases, fish can cause person a severe allergic reaction that leads to anaphylaxis. Fish allergens are parvalbumins, enolases, aldolases, collagen, tropomyosins and vitellogenins [Kleine-Tebbe and Jakob. Springer. 2015, pp. 381-394; Matricardi et al. John Wiley & Sons Ltd. 2016, pp. 173-177]. In 2013 β-enolase (50 kDa) and aldolase (40 kDa) were described as new fish allergens that were purified from Atlantic cod, salmon and tuna. Like parvalbumins, there were found in fish muscle and appeared to be sensitive to thermal treatment. Performed IgE-inhibition ELISA experiment showed the limited inter-species cross-reactivity between discovered β-enolases and aldolases [Kuehn et al. Clinical Et Experimental Allergy. 2013, pp. 811-822]. Currently, there are five β-enolases, extracted from Atlantic cod (Gad m 2), chicken (Gal d 9), striped catfish (Pan h 2), Atlantic salmon (Sal s 2) and Yellowfin tuna (Thu a 2), that are officially recognized as allergens by the World Health Organization/International Union of Immunological Societies.

For fish allergy treatment, the only advice for patients is to avoid eating all kinds of fish, even though some of them could tolerate several fish species. Identifying the type of fish that person is allergic to and the specific allergen is an important step to improve quality of life for an individual patient [Kleine-Tebbe and Jakob. Springer. 2015, pp. 381-94]. Patient, that was allergic to cod and Nile perch, was able to tolerate salmon and tuna, because he was allergic to cod and Nile perch β-enolases and aldolases [Kuehn et al. Letter Ann Allergy Astma Immunol. 2014]. Fish extracts are mixture of proteins, prepared from fish muscle tissue, fillet or from whole fish. These extracts are used in skin prick tests (SPT) for patients to diagnose fish allergy [Ruethers et al. Allergy. 2019, 74, 1352-1363]. However, these natural allergen extracts come with several disadvantages that are related with their poor quality: presence of undefined non-allergenic materials, contaminants, proteases, low allergen concentrations and etc. More detailed characterization and standardized preparation of allergen extract from natural sources could improve its safety and efficacy in allergy diagnostics [Valenta et al. J Allergy Clin Immunol Pract. 2018, 6, pp. 1845-1855].

Hybridoma technology is widely used method to produce monoclonal antibodies (MAbs). The ability of MAbs to recognize and bind to a specific region of antigen, allows their usage in therapy of diseases such as cancer, autoimmune diseases and allergic diseases (asthma and atopic dermatitis) [Landolina and Levi-Schaffer. Br J Pharmacol. 2016, 173(5), pp. 793-803; Chandrasekaran and Sivakumaran. International Journal of Scientific and Research Publications. 2018, 8, pp. 319-338]. Generated MAbs against certain allergen components could be applied in allergen extract standardization by detecting allergen components and by measuring their content in extract. MAbs specific to several *Parietaria judaica* allergen components were used for standardization of *Parietaria judaica* allergenic extracts. Whereas MAbs against Der p 1 and Der f 1 were applied for measurement of these components contents in commercial *Dermatophagoides pteronyssinus* and *Dermatophagoides farinae* extracts respectively [Afferni et al. Biologicals. 1995, 23, pp. 239-247, Chapman et al., J Allergy Clin Immunol. 1987, 80(2), pp. 184-194]. Allergen-specific MAbs could also be used for allergens, like β-lactoglobulin, casein, ovalbumin, lysozyme, parvalbumin, tropomyosin and other, detection in foods, that could provoke life-threatening reactions (anaphylactic shock) for highly sensitized patients [Sharma et al. Food and Drug Administration Papers. 2017, 6, pp. 65-121]. In terms of allergy to fish, polyclonal antibodies against the Atlantic cod paravalbumin were used to develop sandwich ELISA method for fish detection in food [Fæste and Plassen. Journal of Immunological Methods. 2018, 329, pp. 45-55]. Several commercial ELISA Kits are developed for fish parvalbumin quantification in food (like wine, soups, sauces, crackers, surimi and etc.).

Common carp *(Cyprinus carpio)* is one of the most commonly consumed and studied fish species. It was shown that carp parvalbumin contains certain IgE epitopes that are found in various fish species, suggesting that this allergen could be used for *in vitro* and *in vivo* diagnosis of fish allergy [Sharp and Lopata. Clin Rev Allergy Immunol. 2014, 46(3), pp. 258-271, Swoboda et al. J Immunol. 2002, 168, pp. 4576-4584]. Even though parvalbumins are considered as major fish allergens, studies have shown that patients are allergic to other fish allergens: β-enolase and aldolase [Kuehn et al. Clinical Et Experimental Allergy. 2013, pp. 811-822]. Recently common carp β-enolase was officially recognized as allergen and registered under the name Cyp c 2 in WHO/IUIS Allergen Nomenclature database at: http://www.allergen.org/index.php.

There is no information about the development of monoclonal antibodies against common carp β-enolase.Generated MAbs are against human α-enolase [US9382331 B2, publ. 2016], enolase cell surface protein of *Aspergillus fumigatus* [Yadav and Shukla. FEMS Microbiology Letters. 2019, 366], streptococcal surface enolase (SEN) [Fontán et al. J Infect Dis. 2000, 182, pp. 1712-1721], *Plasmodium falciparum* enolase [Pal-Bhowmick et al. J Vect Borne Dis. 2006, 43, pp. 43-52], *Chaetomium globosum* enolase [Green-BJ. MONOCLONAL ANTIBODIES IN IMMUNODIAGNOSIS AND IMMUNOTHERAPY. 2014, 33] and human neuron-specific enolase (NSE, γ-enolase) [Frikke et al. Brain Res. 1987, 417, pp. 283-292; Seshi and Bell CE. Hybridoma. 1985, 4 pp. 13-25].

The above collected information suggests that generation of MAbs against common carp β-enolase would be a useful tool for more detailed allergen characterization and possible application for natural carp and other fish extracts standardization that are used to diagnose fish allergy.

### Summary of the Invention

The aim of the present invention was generation of new hybridoma cell lines producing monoclonal antibodies against common carp β-enolase.

The present invention provides two new hybridoma cell lines titled 14F3 and 6E4 that produce MAbs against β-enolase.These antibodies recognize recombinant common carp β-enolase, fused with maltose binding protein (MBP), expressed in *E. coli.* MAb 6E4 also binds to β-enolase, found in commercial carp and other fish species extracts and in natural carp extract prepared under laboratory conditions.

The present invention provides monoclonal antibodies that recognize amino acid sequence SEQ ID No. 1 
MSISKIHAREILDSRGNPTVEVDLYTAKGRFRAAVPSGASTGVHEALELRDGDKTRYLGKGTQK
AVDHVNKEIAPKLIEKKFSVVEQEKIDKFMLELDGTENKSKFGANAILGVSLAVCKAGAAEKGVP
LYRHIADLAGNKDVILPVPAFNVINGGSHAGNKLAMQEFMILPVGAKNFHEAMRIGAEVYHNLK
 
NVIKAKYGKDATNVGDEGGFAPNILENNEALELLKSAIEKAGYPDKIIIGMDVAASEFFKNGKYDL
DFKSPDDPKRHITGDQLGDLYKSFIKNYPVQSIEDPFDQDDWENWSKFTGSVDIQVVGDDLTV
TNPKRIQQACEKKACNCLLLKVNQIGSVTESIQACKLAQSNGWGVMVSHRSGETEDTFIADLVV
GLCTGQIKTGAPCRSERLAKYNQLMRIEEELGDKAKFAGKDFRHPKL
 or similar sequence, which identity is more than 60%.

The preferred embodiment of the present invention provides the more detailed β-enolase characterization, as an allergen, using newly generated monoclonal antibodies.

Another preferred embodiment provides a use of the monoclonal antibodies of present invention for β-enolase detection in food products or commercial carp extracts.

Finally, the present invention provides a possible application of monoclonal antibodies for creating a diagnostic kit for β-enolase detection in food products or in commercial carp extracts.

The monoclonal antibodies disclosed in the present invention are new and differ from the analogues of the prior art.

The invention is described below in further details and with references to the accompanying drawings and examples.

### Brief Description of Drawings

The generation of new hybridomas and the properties of new monoclonal antibodies are illustrated by Figures 1-7.
Fig. 1 shows picture of PCR clones' analysis.
Fig. 2 shows SDS-PAGE picture of MBP-Eno samples, that were produced in *E. coli* Tuner (DE3) and were checked for the soluble and insoluble cell lysate fractions.
Fig. 3 shows SDS-PAGE picture of purified recombinant MBP-Eno protein, that was purified using MBP Trap HP column prepacked with Dextrin Sepharose HP.
Fig. 4 shows Western blotting picture of recombinant common carp MBP-Eno, recombinant Eno (cleaved from MBP-Eno by TEV protease), recombinant MBP and the lysate of *E. coli* Tuner (DE3) cells. The membrane was developed with monoclonal antibody produced by hybridoma clone 6E4.
Fig. 5 shows Western blotting picture of commercial fish extracts. The membrane was developed with monoclonal antibody produced by hybridoma clone 6E4.
Fig. 6 shows Western blotting picture of recombinant common carp MBP-Eno protein, commercial carp extract (DST, Germany), natural carp extract prepared under laboratory conditions and recombinant MBP protein. The membrane was developed with monoclonal antibody produced by hybridoma clone 6E4.
Fig. 7 shows Western blotting picture of MBP-Eno sample after expression in *E. coli* Tuner (DE3), MBP-Eno-C1 sample after expression in *E. coli* Tuner (DE3), MBP-Eno-C2 sample after expression in *E. coli* Tuner (DE3) and recombinant MBP protein. The membrane was developed with monoclonal antibody produced by hybridoma clone 6E4.

### Detailed Description of the Invention

Recombinant common carp β-enolase, fused with MBP, was expressed in *E. coli,* purified and used for new hybridoma generation. The following procedures were performed by skilled people using approved protocols, that were written according to the previously described hybridoma technology method [Köhler and Milstein. Nature. 1975, 256, pp. 495-497].
1) Immunization - recombinant common carp β-enolase, fused with MBP, in short MBP-Eno, was injected subcutaneously into BALB/c mice. Before the injection, MBP-Eno (antigen) solution was mixed with an equal volume of complete Freund's adjuvant.
2) Four weeks later the mice were injected subcutaneously again with the same dose of the antigen mixed with incomplete Freund's adjuvant.
3) After another four weeks mice were injected with the same dose of the antigen without an adjuvant. The development of new antibodies against the antigen in the sera of immunized mice was monitored by enzyme-linked immunosorbent assay (ELISA) method throughout the immunization.
4) Boost immunization - four weeks after 3^{rd} immunization the selected mouse was injected with the same dose of the antigen without an adjuvant.
5) Three days after boost immunization mouse spleen cells were fused with mouse myeloma Sp 2/0 cells using polyethylene glycol (PEG, Sigma, USA) as a fusion agent.
6) Obtained hybrid cell clones (hybridomas) were tested for their specificity using ELISA method. The aim of this assay was to determine if the hybridomas produce antibodies against β-enolase.
7) The hybridomas were characterized by determining the isotype of produced MAbs and by studying their specificity by ELISA and Western blotting.
8) The hybridomas that produced antibodies against β-enolase were cloned, propagated and frozen for a further storage.
9) Antibodies produced by the hybridomas were characterized by methods:
   a. ELISA and Western blotting using recombinant common carp β-enolase, fused with MBP (MBP-Eno), recombinant Eno (cleaved from MBP-Eno by TEV protease) and recombinant MBP proteins as antigens;
   b. Western blotting using the lysate of *E*. *coli* Tuner (DE3) cells;
   c. ELISA and Western blotting using commercial carp and other fish species extracts as antigens;
   d. Western blotting using carp extract prepared under laboratory conditions;
   e. ELISA and Western blotting using recombinant MBP-Eno, recombinant MBP-Eno-C1, recombinant MBP-Eno-C2 and recombinant MBP proteins as antigens.

Thus, by immunizing BALB/c mice with recombinant MBP-Eno and further fusing spleen cells of the selected immunized mouse with myeloma cells, the hybridomas were generated that produced monoclonal antibodies against β-enolase.It was determined that MAb 14F3 interacts only with recombinant MBP-Eno protein, while MAb 6E4 recognizes native β-enolase from *E*. *coli* bacteria and fish extracts.

Hybridomas 14F3 and 6E4 are characterized by the following features.

*Morphologic properties.* Hybrid cell clones are round-shaped, with a large nucleus. They grow in suspension and are slightly adherent on plastic surface.

*Parent cell cultures.* Hybridomas were obtained by fusion of the immunized BALB/c mouse spleen cells with mouse myeloma cell line Sp 2/0.

*Cell culture properties.* Hybridomas were cultivated under standard conditions. Growth medium is Dulbecco modified Eagl's medium (DMEM) with 2 mM L-glutamine, 200 µg/mL gentamicin and 15% foetal bovine serum. Hybridomas are cultivated in plastic flasks for cell culture. The cells are subcultivated every 3-4 days, 50-100 thousand of cells per millilitre of growth medium. Hybridomas are grown in an incubator in 5% CO₂ atmosphere at 37°C. For a storage, the hybrid cells are frozen in foetal bovine serum containing 10% dimethyl sulfoxide (DMSO) and stored in liquid nitrogen. Freezing conditions: 1 x 10⁶- 2 x 10⁶ cells per millilitre, temperature decrease - 1°C/min until -70°C. After 24 hrs the vials with frozen cells are transferred to liquid nitrogen. For a thawing, the culture is suspended in growth medium without foetal bovine serum at 37°C, centrifuged at 200 g for 5 min and resuspended in growth medium. The viability of cells is 70-80%.

*Contamination.* Hybridoma culture are free of bacteria, fungi and mycoplasma test is negative.

*Productivity of hybridomas.* Monoclonal antibody secretion level in growth medium 10-30 µg/ml. The stability of antibody secretion is up to 5 sub-cultures.

*Characterization of the products.* Hybridomas 14F3 and 6E4 produces MAbs of IgG isotype, IgG2b subtype, specific to common carp β-enolase.

It was found in present invention, that monoclonal antibodies produced by hybridomas 14F3 and 6E4 recognise the following amino acid sequence - SEQ ID No.1.

This amino acid sequence corresponds to amino acid sequence of common carp β-enolase that was determined in Vilnius University Life Sciences Centre Institute of Biotechnology (AWS00995.1; https://www.ncbi.nlm.nih.gov/protein/AWS00995.1).

Other fish species also express β-enolase that contains amino acid sequence that may differ from SEQ ID No.1 by a few amino acid residues.

The amino acid sequence region of common carp β-enolase (SEQ ID No.2), that is recognised by hybridoma 6E4, was specified after additional Western blot experiments with MAb 6E4 and shortened recombinant β-enolase variants (MBP-Eno-C1 and MBP-Eno-C2).

SEQ ID No. 2:

KDVILPVPAFNVINGGSHAGNKLAMQEFMILPVGAKNFHEAMRIGAEVYHNLKNVIKAKYGKDA

TNVGDEGGFAPNILENNEALELLKSAIEKAGYPDKIIIGMDVAASEFFKNGKYDLDFKSPDDPKR

HITGDQLGDLYKSFIKNYPVQSIEDP

Previously described monoclonal antibody anti-EN01 differs from MAbs produced by hybridomas 14F3 and 6E4 as it is specific to human α-enolase [US9382331 B2], whereas MAbs 8304 EB11.6A19L10, 8304 CF5.5L2, 8304 AD9.1L3, 8304 AG4.5A1, 8304 CE9.3A1, 8304 EB11.3A1 and 8304 EF7.6A1 reacts with human neuron-specific enolase. Previously described monoclonal antibody R-5 is against enolase cell surface protein of *Aspergillus fumigatus,* while MAbs 1A10 and 5F3 are against streptococcal surface enolase and react with human α-enolase. Previously described monoclonal antibody 1C7 is specific to *Chaetomium globosum* enolase.

### Embodiments of the Invention

Represented below is information on specific examples on cloning, synthesis in *E. coli* and purification of recombinant common carp β-enolase, fused with maltose binding protein (MBP). Also, information about generation of new MAbs and their properties thereof. These examples and table are presented for illustrative purpose and are not limiting the scope of the present invention.

### Example 1.

### Carp β-enolase cloning and expression in E. coli

Total mRNA was isolated from a carp skeletal muscle using Quick-RNA Miniprep Kit (#R1054, Zymo Research, USA) according to manufacturer's recommendations. The first strand of cDNA was prepared using the RevertAid H Minus First Strand cDNA Synthesis Kit (#K1613, Thermo Fisher Scientific, Lithuania) according to manufacturer's instructions. The cDNA corresponding to the enolase coding gene was amplified using: 5'-GAGATCTATGTCCATCAGTAAGATTCACGCTCG (SEQ ID No. 3) and 5'-CGTCGACTCAGAGTTTGGGGTGGCGGAA (SEQ ID No.4) primers containing BgIII and SalI cleavage sites (underlined), respectively. Polymerase chain reaction (PCR) was performed with the Phusion Flash High-Fidelity PCR Master Mix (F548S, Thermo Fisher Scientific, Lithuania) in a 25µL reaction volume. Amplification reactions included initial denaturation at 98 °C for 10 s, 35 amplification cycles consisting of denaturation for 1 s at 98 °C, annealing for 5 s at 54 °C and extension for 30 s at 72 °C. The PCR fragment of 1316 bp was obtained.

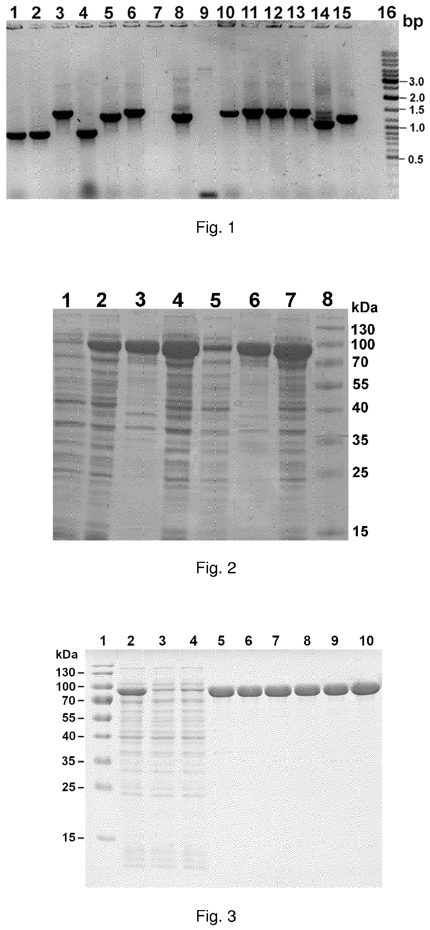

The PCR fragment was cloned into pJET 1.2 vector (K1231, Thermo Fisher Scientific, Lithuania) and the recombinant clones were verified by PCR using primers: 5'-CGACTCACTATAGGGAGAGCGGC (SEQ ID No. 5) and 5'-AAGAACATCGATTTTCCATGGCAG (SEQ ID No.6). Amplification reactions included initial denaturation at 94 °C for 2 min, 30 amplification cycles consisting of denaturation for 30 s at 94 °C, annealing for 20 s at 60 °C and extension for 1 min at 72 °C. The obtained fragments were analysed on 1% agarose gel (Fig. 1).

Fig. 1 shows picture of PCR clones' analysis. Fifteen recombinant clones (lanes 1-15) were subjected for PCR analysis on 1% agarose gel. Lane 16: GeneRuler Ladder mix (SM0331, Thermo Fisher Scientific, Lithuania).

The inserts from five recombinant plasmids (lanes 3, 6, 11, 12 and 13 of Fig. 1) were subjected to sequencing. The clone No.11 sequence (https://www.ncbi.nlm.nih.gov/nuccore/MH255788.1) was closely similar to β-enolase partial sequences published as GenBank: LHQP01000860.1, *Cyprinus carpio* isolate UL-001 Contig859 and GenBank: LHQP01019434.1, *Cyprinus carpio* isolate UL-001 Contig19452. The insert of the clone No.11 has been selected for the expression in *E. coli.*

The DNA fragment plasmid from the recombinant clone No. 11 was excised with BgIII and SalI enzymes and cloned into pET28-MBP-TEV plasmid (#69929, Addgene, USA), cleaved with BamHI and Xhol enzymes. The recombinant clones were verified by restriction and PCR analysis.

The expression plasmid pET28-MBP-TEV-Eno-11 was transformed into *E. coli* Tuner (DE3) strain. The synthesis of β-enolase was induced with 0.1 mM IPTG at OD₆₀₀ = 0.8 and then cells were cultivated for 3-3.5 h at 25 °C and 37 °C. The cells were harvested and disrupted via sonication (Bandelin Sonopuls HD 3100, Bandelin Electronic, Germany). The soluble and insoluble fractions were separated by centrifugation and were analysed on 12% SDS-PAGE under reducing conditions (Fig. 2)

Fig. 2 shows SDS-PAGE picture of MBP-Eno samples, after induction in *E. coli* Tuner (DE3) and the analysis of the soluble and insoluble cell lysate fractions.
Lane 1: lysate of *E*. *coli* cells (OD₆₀₀ = 0.8) before the induction;
Lanes 2 and 5: soluble fraction of cell lysate after induction cultivated at 25 °C and 37 °C, respectively;
Lanes 3 and 6: insoluble fraction of cell lysate after induction cultivated at 25 °C and 37 °C, respectively;
Lanes 4 and 7: total cell lysate after induction cultivated at 25°C and 37°C, respectively;
Lane 8: protein mass standards (#26616, Thermo Fisher Scientific, Lithuania).

### Example 2.

### Purification of recombinant carp β-enolase, fused to maltose binding protein (MBP)

1 g of induced *E. coli* biomass was suspended in 5 ml of 20 mM sodium phosphate buffer, pH 7.4, containing 200 mM NaCl, 1 mM ethylenediaminetetraacetic acid (EDTA) and freshly added 1 mM dithiothreitol (DTT) and 1 mM phenylmethylsulphonyl fluoride (PMSF). The suspension was placed in an ice-water bath and sonicated in pulses of 15 s (total sonication time was 2 min.). The lysate obtained was centrifuged at 20 000 × g for 20 min. The supernatant was diluted 1:6 with 20 mM sodium phosphate buffer, pH 7.4, containing 200 mM NaCl and 1 mM EDTA. Prior to chromatography, the diluted supernatant was filtered through Millex-HV filter with a 0.45 µm hydrophilic PVDF membrane (Merck Millipore, Hertfordshire, UK).

Chromatographic purification was carried out using AKTA purifier 100 chromatography system equipped with the sample pump P-960 and the fraction collector Frac-920 (GE Healthcare Bio-Sciences AB, Uppsala, Sweden). 1 ml MBPTrap HP column prepacked with Dextrin Sepharose High Performance (GE Healthcare Bio-Sciences AB, Uppsala, Sweden) was used. Flow rate was 1 ml/min (0.5 ml/min during sample application). The column was equilibrated with at least 10 CV binding buffer (20 mM sodium phosphate, pH 7.4, 200 mM NaCl, 1 mM EDTA). Clarified lysate containing recombinant carp β-enolase fused to MBP was loaded onto the column followed by washing with at least 10 CV binding buffer. Elution was carried out with 5 CV elution buffer (10 mM maltose in binding buffer). Chromatography fractions were collected.

Total protein concentration in fractions was estimated using the Bradford assay with bovine serum albumin as a standard. Sodium dodecyl sulphate-polyacrylamide gel electrophoresis (SDS-PAGE) of protein-containing fractions was performed on a 12% polyacrylamide gel under reducing conditions. Proteins were stained with PageBlue Protein Staining Solution (24620, Thermo Fisher Scientific, Vilnius, Lithuania).

SDS-PAGE analysis of fractions from MBP-Eno purification is shown in Fig. 3. The fractions eluted with maltose contained one major protein with a molecular mass corresponding to MBP-Eno (91.7 kDa). In the flow-through fractions, the amount of MBP-Eno was low. Most of the protein was retained on the MBPTrap HP column and subsequently eluted with maltose. The total amount of MBP-Eno in eluted fractions was about 5 mg according to Bradford assay from 1g of starting biomass.

Fig. 3 shows SDS-PAGE picture of purified recombinant MBP-Eno protein, that was purified using MBPTrap HP column prepacked with Dextrin Sepharose HP.
Lane 1: protein mass standards (#26616, Thermo Fisher Scientific, Lithuania);
Lane 2: biomass lysate supernatant;
Lanes 3, 4: flow-through fractions;
Lanes 5-10: fractions eluted with 10 mM maltose buffer.

### Example 3.

### Generation of hybridoma cell lines producing monoclonal antibodies against recombinant common carp Eno-MBP

BALB/c mice were immunized with recombinant common carp MBP-Eno (antigen) expressed in *E. coli.* Mice were immunized by injecting subcutaneously 50 µg of the antigen mixed with complete Freund's adjuvant. Four weeks later mice were injected second time with 50 µg of the antigen in incomplete Freund's adjuvant. After another four weeks mice were boosted 3rd time with 50 µg of the antigen without adjuvant. The titres of antibodies specific to the antigen were tested by ELISA throughout immunization. Hybridization was performed three days after the last booster with antigen that was injected for the selected mouse.

Spleen cells of the immunized mouse was fused with mouse myeloma Sp 2/0 cells that lacks hypoxanthine phosphoribosyl transferase, non-proliferates in a selective HAT medium with hypoxanthine-aminopterin-thymidine (10⁻⁴ M hypoxanthine, 1.6 x 10⁻⁵ M thymidine, 4 x 10⁻⁷ M aminopterin) and non-produces immunoglobulins. Cells should be in a logarithmic growth phase before fusion. The fusion was performed by incubation the cells for 5 min with polyethylene glycol solution (PEG-4000) in Dulbecco modified Eagl's medium (DMEM) with 2 mM L-glutamine, 200 µg/mL gentamicin. The ratio of myeloma and spleen cells during fusion was 1:4.5. After fusion, the cells were washed with DMEM containing 15% foetal bovine serum, resuspended in a selective HAT medium and seeded into 96 well plates at a density 2.6 x 10⁵ cells per well. Hybrid clones appeared on days 5-10 after fusion. Growth medium was tested by an indirect ELISA for antigen specific antibodies. One-half of the growth medium was changed every 4-5 days. For a first time HAT medium was changed to HT medium containing hypoxanthine-thymidine (10⁻⁴ M hypoxanthine and 1.6 x 10⁻⁴ M thymidine). After several days HT medium was changed stepwise to normal growth medium. Hybrid clones producing antibodies specific to MBP-Eno were cloned by limiting dilution assay. The clones appeared on days 4-7 after cloning and were tested by an indirect ELISA. Selected clones were propagated, then cultivated *in vitro* or frozen for storage in liquid nitrogen. Two hybridoma cell lines producing monoclonal antibodies against MBP-Eno were designed 14F3 and 6E4.

### Example 4.

### Determination of the specificity and isotypes of monoclonal antibodies

ELISA and Western blotting methods were applied to determine the specificity of antibodies produced by clones 14F3 and 6E4. Antibodies isotypes were determined by commercial isotyping test kit (Bio-Rad, USA).

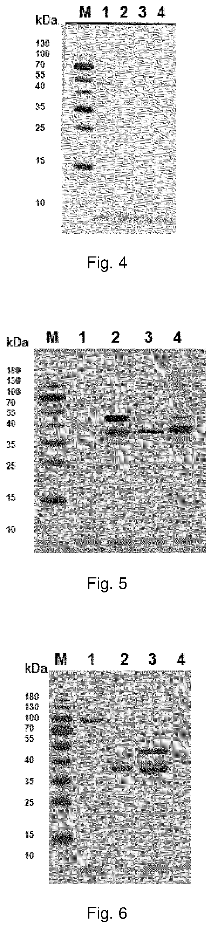

Using these assays, it was identified that MAbs are of IgG isotype, IgG2b subtype and recognize recombinant β-enolase (Fig. 4). By using Western blotting it was shown that monoclonal antibody 6E4 reacts with 50 kDa protein in the lysate of *E.coli* Tuner (DE3) cells that corresponds to β-enolase according to its molecular weight (Fig. 4).

Therefore, obtained monoclonal antibodies are specific to β-enolase.

The data on the specificity and isotypes of monoclonal antibodies produced by hybridomas 14F3 and 6E4 are summarized in Table 1.

| Hybridoma clone | Subtype (subclass) | Reactivity with different antigens | | | | | |
|---|---|---|---|---|---|---|---|
| | | ELISA | | Western blotting | | | |
| | | Eno-MBP | MBP | Eno-MBP | Eno | MBP | *E. coli* lysate |
| 14F3 | IgG2b | + | - | - | - | - | - |
| 6E4 | IgG2b | + | - | + | + | - | + |

Fig. 4 shows Western blotting picture, where recombinant common carp MBP-Eno, recombinant Eno (cleaved from MBP-Eno by TEV protease), recombinant MBP and *E. coli* Tuner (DE3) lysate were immunostained with monoclonal antibody produced by hybridoma 6E4.
Lane 1 - recombinant purified Eno protein;
Lane 2 - recombinant purified MBP-Eno protein;
Lane 3 - recombinant purified MBP protein;
Lane 4 - cell lysate from *E. coli* Tuner(DE3);
M - protein mass standards (#26616, Thermo Fisher Scientific, Lithuania).

For more detailed characterization of monoclonal antibody, secreted by hybridoma 6E4, the amino acid sequences of the antibody heavy and light chains were determined (Synbio Technologies, USA).

The immunoglobulin heavy chain (IgH) amino acid sequence (SEQ ID No.7):

QVQLQQSGAELVRPGASVTLSCKASGYTFIDYEMHWVKQTPVHGLEWIGAIDPETGGTAYNQ
KFKGKAILTADKSSSTAYMELRSLTSEDSAVYYCSPTLQGDYWGQGTTLTVSS

The immunoglobulin light chain (IgL) amino acid sequence (SEQ ID No. 8):

DVVMTQTPLSLPVSLGDQASISCRSSQSLVHSDGNTYLYWYLQKPGQSPKLLIYKVSNRFSGV
PDRFSGSGSGTDFTLKISRVEAEDLGVYFCSQSTHIPWTFGGGTKVEIK

### Example 5.

### Investigation of the cross-specificity of monoclonal antibodies

By using ELISA and Western blotting the hybridoma clone 6E4 and clone 14F3 were tested for their cross-specificity, i.e., their reactivity with carp and other fish species commercial extracts (DST, Germany).

This investigation has shown that MAb 14F3 reacts only with salmon extract, while MAb 6E4 with all fish extracts (Table 2, Fig. 5). It demonstrates that both MAb 14F3 and MAb 6E4 are specific not only to recombinant common carp β-enolase, but also recognize other fish species β-enolases.

The data on the specificity of monoclonal antibodies produced by hybridomas 14F3 and 6E4 with different fish extracts are summarized in Table 2.

| Hybridoma clone | Reactivity with different antigens | | | | | | | |
|---|---|---|---|---|---|---|---|---|
| | ELISA | | | | Western blotting | | | |
| | Extracts | | | | | | | |
| | Cod | Carp | Herring | Salmon | Cod | Carp | Herring | Salmon |
| 14F3 | - | - | - | + | - | - | - | - |
| 6E4 | + | + | + | + | + | + | + | + |

Fig. 5 shows Western blotting picture of commercial fish extracts. The membrane was developed with monoclonal antibody produced by hybridoma clone 6E4.
Lane 1 - commercial herring extract (f21, DST, Germany);
Lane 2 - commercial salmon extract (f41, DST, Germany);
Lane 3 - commercial carp extract (f233, DST, Germany);
Lane 4 - commercial cod extract (f3, DST, Germany);
M - protein mass standards (#26616, Thermo Fisher Scientific, Lithuania).

By using Western blotting hybridoma clone 6E4 was tested for applicability to detect β-enolase in natural carp extract, that was prepared in Vilnius University Life Sciences Center Institute of Biotechnology Department of Immunology and Cell Biology laboratory. Extract was extracted from carp fillet (∼ 4 g) using phosphate buffered saline (pH 7.4), containing 0.15 M NaCl.

It was shown that MAb 6E4 reacts with β-enolase contained both in newly prepared extract and in commercial carp extract (f233, DST, Germany) and could be applied for the characterization of carp extracts from different manufacturers.

Fig. 6 shows Western blotting picture of recombinant common carp MBP-Eno protein, commercial carp extract (DST, Germany), natural carp extract prepared under laboratory conditions and recombinant MBP protein. The membrane was developed with monoclonal antibody produced by hybridoma clone 6E4.
Lane 1 - recombinant purified MBP-Eno protein;
Lane 2 - commercial carp extract (f233, DST, Germany);
Lane 3 - extract prepared from carp fillet;
Lane 4 - recombinant purified MBP protein;
M - protein mass standards (#26616, Thermo Fisher Scientific, Lithuania).

### Example 6.

### Determination of the epitope, that is recognised by hybridoma clone 6E4 secreted monoclonal antibody.

By using ELISA and Western blotting the hybridoma clone 6E4 was tested for its specificity for shortened recombinant β-enolase variants (MBP-Eno-C1 and MBP-Eno-C2). Recombinant proteins were produced in *E. coli* Tuner (DE3) bacteria like recombinant MBP-Eno protein. All synthesis was performed in Vilnius University Life Sciences Centre Institute of Biotechnology Department of Immunology and Cell Biology laboratory.

MBP-Eno (836 amino acids) protein sequence corresponds to SEQ ID No. 9.

MBP-Eno-C1 (698 amino acids) is shortened recombinant MBP-Eno protein variant (shortened C-terminus of MBP-Eno protein). Protein sequence corresponds to SEQ ID No. 10.

MBP-Eno-C2 (543 amino acids) is shortened recombinant MBP-Eno protein variant (shortened C-terminus of MBP-Eno protein). Protein sequence corresponds to SEQ ID No. 11.

After performing Western blotting, it was determined, that MAb 6E4 reacts with recombinant MBP-Eno and recombinant MBP-Eno-C1 proteins, but no reactivity was shown with recombinant MBP-Eno-C2 protein.

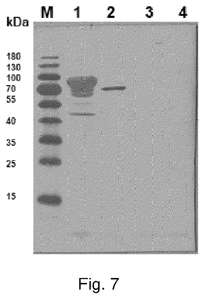

Fig. 7 shows Western blotting picture of MBP-Eno sample after expression in *E. coli* Tuner (DE3), MBP-Eno-C1 sample after expression in *E. coli* Tuner (DE3), MBP-Eno-C2 sample after expression in *E. coli* Tuner (DE3) and recombinant MBP protein. The membrane was developed with monoclonal antibody produced by hybridoma clone 6E4.
Lane 1 -MBP-Eno sample after induction;
Lane 2 - MBP-Eno-C1 sample after induction;
Lane 3 - MBP-Eno-C2 sample after induction;
Lane 4 - recombinant purified MBP;
M - protein mass standards (#26616, Thermo Fisher Scientific, Lithuania).

Comparison of the amino acid sequences of all recombinant proteins (Scheme 1) revealed the amino acid sequence of β-enolase (544-698 amino acids), which is recognized by MAb 6E4 and is thought to be antibody epitope (SEQ ID No. 12).

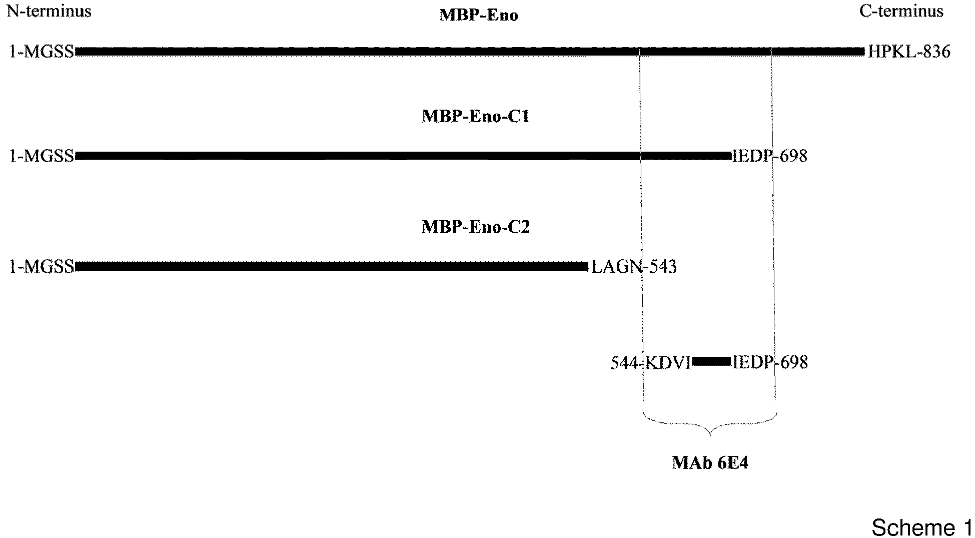

## Claims

1. A monoclonal antibody, **characterized in that** it recognizes an amino acid sequence SEQ ID No. 1 or a similar sequence, which identity is at least 95%.

2. A hybridoma cell line 14F3, producing monoclonal antibodies according to claim 1, deposited at BCCM with accession number LMBP 13806CB.

3. A hybridoma cell line 6E4, producing monoclonal antibodies according to claim 1, deposited at BCCM with accession number LMBP 13805CB.

4. A hybridoma cell line, selected from a group consisting of 14F3 and 6E4, producing monoclonal antibodies as defined in claim 1 against common carp β-enolase.

5. A monoclonal antibody according to claim 1, produced by hybridoma cell line according to any one of claims 2-3, which is an IgG antibody.

6. The monoclonal antibody according to claim 5, which is a murine monoclonal antibody.

7. Use of the monoclonal antibody according to claim 1 or claim 5 for β-enolase detection in food products or in commercial carp extracts.

8. A diagnostic kit for the detection of β-enolase in food products or in commercial carp extracts, comprising at least a monoclonal antibody according to claim 1 or claim 5.
